# EUROPEAN PATENT SPECIFICATION

(11) **EP 3 010 693 B1**
(45) Date of publication and mention of the grant of the patent: **28.10.2020**
(21) Application number: 14838102.3
(22) Date of filing: 03.07.2014
(51) Int. Cl.: B27L 11/02, G01N 33/46, F23K 1/00, C10L 5/44, C10L 5/36, C10L 5/14, B30B 11/00, B27M 1/08, B27L 11/00

(54) **METHOD FOR MONITORING, SAMPLING AND CONTROL OF WOOD PELLET MANUFACTURING**
VERFAHREN ZUR ÜBERWACHUNG, PROBENAHME UND STEUERUNG DER HERSTELLUNG VON HOLZPELLETS
PROCÉDÉ DE CONTRÔLE, D'ÉCHANTILLONNAGE ET DE COMMANDE DE FABRICATION DE GRANULÉS DE BOIS

(30) Priority: 20.08.2013 SE 1350961
(43) Date of publication of application: 27.04.2016
(73) Proprietor: Uny-Konsult AB, 57082 Målilla (SE)
(72) Inventor: NYGREN, Ulf, S-57082 Målilla (SE)
(74) Representative: Bergenstråhle Group AB
(86) International application number: PCT/SE2014/050846
(87) International publication number: WO 2015/026280

(56) References cited:
- EP-A1- 0 266 403
- EP-A1- 2 045 057
- US-A1- 2006 278 353
- US-A1- 2007 089 805
- Jaya Shankar ET AL: "A Review on Biomass Densification Technologies for Energy Application", , 31 August 2010 (2010-08-31), XP055606620, Retrieved from the Internet: URL:https://pdfs.semanticscholar.org/305d/ 4fb8d8c782e40caa96847d555e02159b9c74.pdf [retrieved on 2019-07-17]

## Description

### Technical field

The present invention relates to a method for monitoring, sampling and control of a plant for producing wood pellets.

### Background of the invention

With the development in the energy and environment sectors, the need for wood pellets has increased. Not least in the private market through the installation of pellet burners for heating of households and domestic hot water, the development has been rapid and the need for producing pellets of good quality has increased.

Wood pellets can be divided into so-called "industry pellets" and so-called "consumption pellets" for private use. At wood pellet manufacturing there are different quality standards to follow-up and control the quality of the produced wood pellets. For production of good quality consumption pellets for private use, the European norm SS-EN 14961 - 2:201 1, is available. This standard prescribes for the A1 -class, two important limits:
1. The moisture content in the final product, i.e. the completed wood pellets, shall be less than 10%.
2. The bulk density should be higher than 600 kg/m³.

However, the client requirements are in most cases harder than that, why the industry strives for a good pellet with high quality by achieving:
1. The moisture content in the final product, i.e. the completed wood pellets, shall be less than 8% ± 0.5%.
2. The bulk density should be higher than 630 kg/m³.

At the simplest wood pellet plants are often pellets produced of a lower and often uneven quality.

In these plants, samples are taken at the produced wood pellets, which samples then are sent to a testing institute for verification of quality and thus the wood pellets are expected to maintain the tested quality. The wood pellets can after tuning of production of course achieve a good quality and a quality certificate may outwardly give the appearance of good wood pellets, but since the quality is dependent, among other things, of the raw wood material that is fed into the plant, the quality of the end product shifts, unless careful monitoring and control is performed. Thus the possibility exists that inferior wood pellets can be sold as a higher quality than the actual produced wood pellets have at the moment. At plants that produce wood pellets with high and consistent quality, a continuous sampling and control must be performed to ensure consistent production quality. It is attractive for wood pellet manufacturers to deliver pellets with so-called Nordic Ecolabel. The Nordic Ecolabel is a famous Swedish official ecolabel with a large number of harsh environmental, health and climate requirements. Among other things, this requirement demands a dense sampling and documentation of the quality of the end product - the final wood pellets product.

At today's wood pellet factories the plant is controlled, so to speak, "from the front", that is, to control and monitor the wood raw material - the wood chip material - in respect of the correct moisture content, for example, at the entrance to the wood pellet press. The measurement method used in connection with this is today usually the use of moisture sensors with capacitive measurement, which method of course can be performed continuously during operation, but the disadvantage is that one has to tune the sensors depending on the type of raw material to be measured - sawdust or chips - whereby an assessment of the operation that will run, takes place, i.e. control "from the front". Additionally the admixture of starch or similar is controlled depending of the wood material entering the facility, as well as the input to the pellet press.

Further, at certain intervals, samples are taken manually on the produced wood pellets, regarding moisture content and bulk density. This is done by that a certain amount of wood pellets is taken out and being ground in a mill, whereafter the moisture content is measured by a technique known as NIR, which measures mainly surface moisture, and the measurement is done with a manual measuring instrument. These values are documented and with this information, the operator makes the adjustments that he or she considers necessary in the various process steps in the production line, in order to make the final product eventually get a quality level that exceeds set limits on the bulk density and moisture content. How the change of the control parameters affected the final result will be visible only at the next manual sampling and evaluation, in the best case, after some hours, whereafter new adjustment eventually are made. This process is time and resource intensive and requires high skills of the operator. While a team of a larger facility may consist of 3-4 people and the operation continues around the clock in three shifts, this means a large number of operators who all must have high and consistent level of competence in order to obtain a consistent quality of the final product. Especially as the sampling frequency according to high quality standards takes place approximately once per hour, the work load is high, and therefore it is not always economical to have such dense sampling, required given the personnel costs, etc., to obtain a high quality for example according to the Nordic Ecolabel.

In addition, the quality can still vary depending on the staff's assessments and competency, and that the wood raw material fed into the facility generally varies, which then must constantly be taken into account by the operating personnel. Wood raw material plays a big role in the end result - pine or spruce, fresh or old chips, fresh or old pulpwood etc. - why it is not always easy to make the right decisions based on input and manual evaluation of the sample every now and then. Furthermore, there are tolerances of the dryers, which makes sawdust moisture content continuously vary if the temperature of the dryer varies. Thus, the quality of the wood pellets can vary during periods of the production day.

A known method for controlling a fodder mixing plant for producing of fodder pellets is disclosed in EP 0 266 403 A1. The fodder pellet plant comprises more or less similar equipment as used within wood pellet manufacturing, for example dosing device for additives, mixing device, humidity adjusting device, pellet press, dosing screw, cooling device etc. The method includes sampling performed on the finalized fodder pellets but some steps in the process of fodder pellets differs from the steps of producing fodder pellets. For example, the raw material fed into a fodder pellet process differs from the raw material fed into a wood pellet process where wood raw material is fed to the process in the form of chip raw material. The chip raw material differs "from day to day" and even from one hour to another according to density, moisture content, wood type etc., and therefore it is of great importance in a wood pellet process to for example analyze the chip raw material fed into the process. This among other steps is not applicable to a fodder pellet process.

In the report "A Review on Biomass Densification Technologies for Energy Application", 31 August 2010, by Jaya Shankar ET AL, a normal wood pellet process and how to treat the wood material in different steps is disclosed. The report describes more or less a process which is controlled "from the front" as mentioned above.

### Disclosure of the invention

The challenges of the prior art are achieved by a method according to claim 1. Additional advantageous features appear from the dependent claims 2-7.

With the present invention the object is achieved to solve the above described problems, and also bring forward the technique within the art. The new and now present method relates both to streamline running operations, but above all ensure a high and consistent quality of the wood pellets by that the sampling and analysis takes place on the final product, i.e. the wood pellets. In addition, the sampling, the control measurement of predefined parameters, the evaluation of the test results, and - dependent on test results - possible adjustment of appropriate control parameters, takes place almost continuously according to a predetermined frequency and also fully automated during the operation of the facility, in order to achieve the desired level of quality of the wood pellets. When the control of the end product is done, so to say "online", and the result is continuously evaluated against predefined set points, for that particular quality that is desired, the appropriate control parameters are adjusted in principle immediately, and optimization continues thus continuously without long periods of time between the actions, as in older solutions. Furthermore, the process is not dependent on the individual skill of the operator, but the process follows the preprogrammed routines for adjustment of control parameters in the steps that are proven suitable for different scenarios. Thus obtained no long periods of poorer quality of the wood pellets and the highest quality standards are thereby achieved, which present plants for wood pellets production cannot achieve, particularly because of the manual handling. The manual monitoring and management according to current technology also means that operating costs are significantly higher than using the method of the present invention. According to the inventive method a new type of sensor with microwave technology, is used, which makes it possible to, in principle continuously, measure the moisture content of the completed pellets. Older types of sensors for continuous measurement, measures according to capacitive technology, which prevents reliable measurement of moisture content in the finished pellets, since the capacitance is dependent on the bulk density. As described above, one has so far taken a sample of the finished wood pellets, grinded the pellets in a mill and analyzed the sample with the so-called NIR method. The instruments currently used for this purpose are usually hand-held instruments that are very expensive. By using microwave detectors in the testing of the final product a cost-effective, reliable and monitorable way is obtained for collecting data from the production flow, without the involvement of manual intervention, and control the quality on the basis of the final product, so to speak control the process "from the back."

As the use of wood pellets may vary (industrial/private, etc.), and that a purchaser of wood pellets can have different demands on quality, can, according to a preferred embodiment of the method, the set points for respective control parameter be adjusted so that the final product quality is controlled by the frequent checks and through adjusting the control parameter/parameters towards the selected set point and gets the desired quality of wood pellets for the current production order - the quality is controllable and selectable through adjustable setpoint control parameters. Further advantages of adjustable setpoints are while, as mentioned earlier, the quality/composition of the raw material in the form of sawdust input to the facility varies, then the setpoints are also adjustable based on these data. The setpoints are adjusted based on the measurement of the final product as stated above, or a combination of input and output.

Depending on the chosen quality level of the final product, and controlled by the particular standard that determines how often the sampling and documentation shall be made of the final wood pellet product, is the sampling frequency, according to a preferred embodiment of the new method, controllable by that the time between samples can be changed. Within the scope of the invention the time is freely selectable and optimized based on what is practically possible in the process and depending on the time it takes to convert one or more control parameters and make an impact in the process. At today's plants with manual testing, documentation and parameter control, the sampling frequency is strongly limited by the cost and time aspect, and takes place at best one time per hour, at the highest quality of wood pellets. In a plant with control according to the now present method, it is no problem to have as frequent sampling intervals as every 15 minutes.

When measuring the bulk density - a manual test is made according to today's technology, by taking up 3 liters of pellets and weigh it on a scale. In the present method, a sample cylinder is automatically filled with pellets from process, when sampling is initiated. This sample cylinder is suspended from weighing cells/measuring cells which records the weight of the sample and sends an output signal dependent on the weight, and the output is recorded and then processed, for example, adjusting the control parameter for the dosing screw to the pellet press or other control parameter. By that this part of the sampling also is performed on the final product and automatically, the process is not operator-dependent, unlike earlier solutions.

In the method, the control measurement of the moisture content in the finished wood pellets is made in connection with the control measurement of bulk density. This is done automatically by initiating sampling, whereby the sample cylinder is filled automatically as described above by measuring the bulk density.The sample that is collected by the sample cylinder is also analyzed regarding moisture content, using the microwave detector. By combining moisture measurement with volume measurement, which is automatic, even the moisture measurement becomes automatic and also representative, by measuring continuously the same amount of wood pellets - that is 3 liters/sample. Automatic moisture measurement on finished wood pellets is not at hand in current plants, only automatic moisture measurement of the saw dust material.

The automatic sampling with using the sample cylinder according to above further enables that the same sample is analyzed regarding temperature, which is performed according to the method. The temperature is measured by a temperature sensor arranged at the sample cylinder and an automatic and representative measurement and analysis of the temperature of the wood pellets in this process step is thereby obtained.

In a further preferred embodiment a computer software is used for the manufacturing process, which software is designed specifically for the new method. The software controls the sampling frequency, sampling, control measurement orders for the sensors regarding moisture content, weight, temperature, collection of test results, analysis of results, comparison to the programmed set points of the control parameters for moisture content, weight and temperature and if necessary adjusting the control parameters to achieve the desired quality. Examples of control parameters that affect the process are change of moisture content of sawdust raw material at the belt dryer, control of dosing screw (increase/decrease) for the incorporation of starch in the chip, change the moisture content of the chip at a mixer arranged after the starch incorporation device, changing the flow of material into the pellet press through a metering screw providing chips to the press as well as affect the rotation speed of the pellet press for determining the toughness and hardness of the finished pellets. Today there is no complete system that includes a whole grip of this process, including the evaluation of finalized end product.

In a further preferred embodiment of the method, the entire process is remotely controllable by the computer software, which is why a crane operator who manages the loading of wood raw material, or an operator, on-site or remotely, receives ongoing information about the process of elective degree. There are, for example "online operating data" and quality information about the final product, alarms, etc. Many municipalities are currently building cogeneration plants of various kinds, which are connected to a district heating network. A common "problem" is in the summer when there is not enough load, hence it is advantageous to use the waste heat, for example for drying wood chips to a wood pellet facility. Through the remote control there is no dependency on the physical location of the plant itself, which up till now has been the case. Furthermore, one is not depending on that the operators are on site for sampling, monitoring and control. As mentioned before, according to today's technology one is dependent of that the operation staff is on site around the clock, for monitoring, adjustment and control.

In a preferred embodiment, the documentation of test results and the evaluation of wood pellet quality is made automatically and continuously to thereby achieve the highest quality standards for the wood pellets. As the entire sampling, monitoring, and control process is automatic, it is also possible to log the desired data to the extent necessary for that pellet quality which is currently produced in the plant. This makes the wood pellets both documented in the appropriate quality and thus quality assured and also traceable.

Through the invention, a number of advantages over known solutions are obtained:
- Control based on the end product - the finalized wood pellets.
- A fully automated plant.
- Direct tuning of the pellet quality if needed.
- Frequent sampling is not cost driving.
- The pellet quality is not operator dependent.
- Controllable quality through controllable set point values.
- Computer control enables storage of operational data on which the control is based. Also enables remote operation.

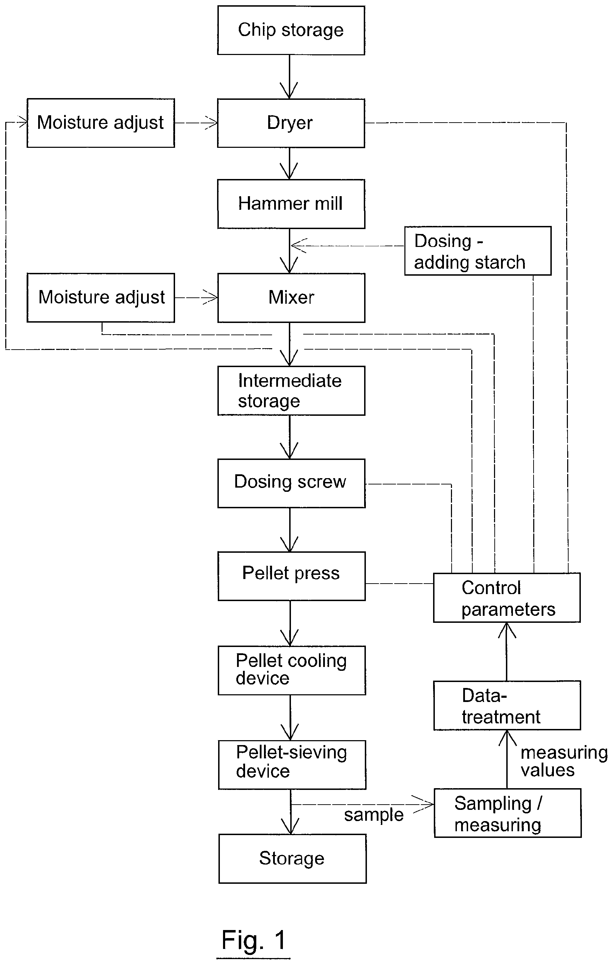

### Short description of figure 1

The present invention is explained through a non-limiting example with reference to the attached drawing, which shows a block diagram of the manufacturing process and its method of monitoring, testing and controlling. Included components are not presented in detail but can vary depending on the nature of the plant and scope. The invention moves the state of the art within this technical field further in various aspects. This is realized in the present invention by a method of the type described below.

### Detailed description

**Fig.1** shows a principled view of the most important parts of the plant in the form of a block diagram. A plant for the manufacture of wood pellets can vary greatly in terms of size, component parts and processes. Among others, there are different storages, intermediate storages, retrieval devices for chip spillage and dust for recycling into the process or for manufacture of other wood products, fire protection devices, etc. All such things have in Figure 1 been omitted to include only the most important parts for the description of the method of the invention.

The raw material in the form of wood chips can vary greatly in terms of both the wood (pulpwood) coming into the facility and that it also can be recycled wood materials that go into feedstock. The woods species contains different amounts of lignin and moisture and naturally recycled materials can also vary in respect of moisture content.

Chip raw material is first arriving to a dryer, preferably a belt dryer, where much of the moisture in the material is removed. As previously stated, the goal of the completed pellet is a moisture content between 8-10%. The dryer is controlled by temperature and moisture content in the chip is normally measured continuously with capacitive sensors. At the dryer moisture is added if necessary. From the dryer the chips are further fed to a so-called hammer mill, where the chips are milled further, compared with the previously termed chip raw material, and this to become an appropriate size for the pellet press. From the hammer mill the chips are further fed in the direction to a mixer, and before the mixer they pass a dosing device for additives. The dosing device here consists of a so called dosing screw.

Typically, the raw material contains lignin, but this varies by wood species, grade of recycled materials and so on, so an additive in the form of starch may be needed to obtain a good final product. In the subsequent mixer the chips and additive are then mixed, and in the mixer it is also possible to adjust the moisture content of the chips when necessary. Then, the chips are fed further to an intermediate storage from which the chips via a controllable dosing screw are fed into the pellet press. By changing the feed rate of the chips to the pellet press the properties of the pellets is affected. Additionally it is possible to control the rotational speed of the pellet press for further tuning of the properties. The finalized wood pellets now move on to a cooling device for cooling of the pellets and after the cooling, the final product is sifted in a pellet-sieving device, where leftovers of chips and dust is collected and recycled into the facility. After the pellet-sieving device sampling takes place automatically according to the method, after the initiation of sampling from the software, whereby a sample cylinder is automatically filled with wood pellets. The sample is analyzed regarding moisture content using one or more microwave sensors, regarding temperature by a temperature sensor and also regarding bulk density by that the sample cylinder is arranged at a measuring cell for measuring the volume weight of the sample. In the figure, none of the details is shown but mentioned only in general with "test", "sampling/measurement" and "measurement". The measurement values are treated in the specially designed software and are compared against defined quality/control parameters, and based on experience from previous tuning and past production history, control signals are sent for the control parameters used in the predetermined control algorithms for each operating condition and for each desired quality. As seen in the figure by the dashed lines, it is possible to affect drying temperature, the moisture content of wood chips at the dryer, the dosing of additives, moisture addition in the mixer, the feeding of material with the dosing screw to the pellet press, and the rotation speed of the pellet press. Of course concept of the invention is not limited only to the above control parameters but also other parts of the plant may as well be controllable. The key is that sampling is done on the final product and that sampling, testing measurement and adjustment of control parameters is done automatically and at frequent intervals without depending on the individual operator skill and decisions based on the specific moment, based on a manual sample. By the development of the included testing and measurement components for automatic operation and quality assured pellets a technological leap has been taken in the wood pellet manufacturing business.

## Claims

1. Method for monitoring, sampling and control of a plant for producing wood pellets, the method comprising:
- receiving chip raw material in a chip dryer in which moisture is removed from the material, wherein the chip dryer is controlled by temperature and in which chip dryer moisture content in the chips is measured continuously with capacity sensors and adjusted if necessary by adding moisture,
- from the chip dryer further feeding the chips to a hammer mill in which the chips are milled further compared with the chip raw material,
- from the hammer mill further feeding the chips in direction to a mixer passing before the mixer a dosing device for additives in which additives may be added,
- in the subsequent mixer the chips and additive are mixed, wherein in the mixer the moisture content may be adjusted by a humidity adjusting device,
- from the mixer further feeding the chips to an intermediate storage,
- from the intermediate storage feeding the chips by a dosing screw for a pellet press to a pellet press in which wood pellets are produced,
- moving these finalized wood pellets to a pellet cooling device,
- after the cooling sifting the finalized wood pellets in a pellet-sieving device,
- after the sifting automatic sampling of the wood pellets by automatically filling a sample cylinder with wood pellets, and
- analyzing the sample regarding moisture content by using one or more microwave sensors, regarding temperature by using a temperature sensor and regarding bulk density by that the sample cylinder is arranged at a measuring cell for measuring the volume weight of the sample, wherein the sampling, control measurement of the sample regarding predefined control parameters, evaluation of the test results, possible adjustment of appropriate control parameter based on the test results, are performed according to predetermined frequency and fully automated during operation of the plant, to control the quality level of the wood pellets.

2. Method according to claim 1, **characterized by** that a setpoint for each control parameter is adjustable, to control the quality of the wood pellets.

3. Method according to claim 1 or 2, **characterized by** that the sampling frequency is adjustable.

4. Method according to any of the preceding claims, **characterized by** that the measuring cell delivers an output signal dependent on the sample weight.

5. Method according to any of the preceding claims, **characterized by** that monitoring, sampling, control measurement and adjustment of control parameters is made by means of computer software.

6. Method according to claim 5, **characterized by** that the computer software is remotely controlled.

7. Method according to any of the preceding claims, **characterized by** that the documentation of the pellet quality is done automatically during the control measurement, for securing that the produced wood pellets fulfil the desired quality.

## Patentansprüche

1. Verfahren zur Überwachung, Probenahme und Steuerung einer Anlage zum Herstellen von Holzpellets, wobei das Verfahren umfasst:
- Aufnehmen von Spanrohmaterial in einem Spantrockner, in dem Feuchtigkeit aus dem Material entfernt wird, wobei der Spantrockner temperaturgesteuert ist und wobei in dem Spantrockner ein Feuchtigkeitsgehalt in den Spänen fortlaufend mit kapazitiven Sensoren gemessen und bei Bedarf durch Hinzufügen von Feuchtigkeit angepasst wird,
- von dem Spantrockner, weiter Zuführen der Späne zu einer Hammermühle, in der die Späne im Vergleich zu dem Spanrohmaterial weiter zerkleinert werden,
- von der Hammermühle, weiter Zuführen der Späne in Richtung eines Mischers, wobei sie vor dem Mischer eine Dosiervorrichtung für Zusatzstoffe passieren, in der Zusatzstoffe hinzugefügt werden können,
- in dem nachfolgenden Mischer, Mischen der Späne und Zusatzstoffe, wobei in dem Mischer der Feuchtigkeitsgehalt durch eine Feuchtigkeitsanpassungsvorrichtung angepasst werden kann,
- von dem Mischer, weiter Zuführen der Späne zu einem Zwischenspeicher,
- von dem Zwischenspeicher, Zuführen der Späne durch eine Dosierschnecke für eine Pelletpresse zu einer Pelletpresse, in der Holzpellets hergestellt werden,
- Bewegen der fertiggestellten Holzpellets zu einer Pelletkühlvorrichtung,
- nach dem Kühlen, Sieben der fertiggestellten Holzpellets in einer Pelletsiebvorrichtung,
- nach dem Sieben, automatische Probenahme der Holzpellets durch automatisches Füllen eines Probenzylinders mit Holzpellets, und
- Analysieren der Probe hinsichtlich eines Feuchtigkeitsgehalts mithilfe eines oder mehrerer Mikrowellensensoren, hinsichtlich einer Temperatur mithilfe eines Temperatursensors und hinsichtlich einer Rohdichte, indem der Probenzylinder an einer Messzelle zum Messen des Raumgewichts der Probe angeordnet wird, wobei die Probenahme, Steuermessung der Probe hinsichtlich vorbestimmter Steuerparameter, Auswertung der Prüfergebnisse, eventuelle Anpassung der entsprechenden Steuerparameter basierend auf den Prüfergebnissen gemäß einer vorbestimmten Häufigkeit und vollautomatisiert während eines Betriebs der Anlage durchgeführt werden, um das Qualitätsniveau der Holzpellets zu steuern.

2. Verfahren nach Anspruch 1, **dadurch gekennzeichnet, dass** ein Sollwert für jeden Steuerparameter anpassbar ist, um die Qualität der Holzpellets zu steuern.

3. Verfahren nach Anspruch 1 oder 2, **dadurch gekennzeichnet, dass** die Probenahmehäufigkeit anpassbar ist.

4. Verfahren nach einem der vorhergehenden Ansprüche, **dadurch gekennzeichnet, dass** die Messzelle ein Ausgangssignal in Abhängigkeit von dem Probengewicht liefert.

5. Verfahren nach einem der vorhergehenden Ansprüche, **dadurch gekennzeichnet, dass** die Überwachung, Probenahme, Steuermessung und Anpassung von Steuerparametern mithilfe von Computersoftware vorgenommen wird.

6. Verfahren nach Anspruch 5, **dadurch gekennzeichnet, dass** die Computersoftware ferngesteuert ist.

7. Verfahren nach einem der vorhergehenden Ansprüche, **dadurch gekennzeichnet, dass** die Dokumentation der Pelletqualität automatisch während der Steuermessung durchgeführt wird, um sicherzustellen, dass die hergestellten Holzpellets die gewünschte Qualität erreichen.

## Revendications

1. Procédé de surveillance, d'échantillonnage et de contrôle d'une installation de fabrication de granulés de bois, le procédé comprenant :
- la réception de matière première en copeaux dans un séchoir à copeaux où l'humidité est éliminée de la matière, le séchoir à copeaux étant régulé en température et la teneur en humidité des copeaux du séchoir à copeaux étant mesurée en continu à l'aide de capteurs capacitifs et ajustée au besoin par apport d'humidité,
- à partir du séchoir à copeaux, l'acheminement ensuite des copeaux jusqu'à un broyeur à marteaux où les copeaux sont davantage broyés par comparaison à la matière première en copeaux,
- à partir du broyeur à marteaux, l'acheminement ensuite des copeaux en direction d'un mélangeur en les faisant passer, avant le mélangeur, par un dispositif de dosage d'additifs où des additifs sont susceptibles d'être ajoutés,
- le mélange, dans le mélangeur qui suit, des copeaux et des additifs, la teneur en humidité dans le mélangeur étant ajustable par un dispositif d'ajustement d'humidité,
- à partir du mélangeur, l'acheminement ensuite des copeaux jusqu'à un stockage intermédiaire,
- à partir du stockage intermédiaire, l'acheminement des copeaux, par une vis doseuse pour une presse à granulés, jusqu'à une presse à granulés où des granulés de bois sont fabriqués,
- le transfert de ces granulés de bois finaux jusqu'à un dispositif de refroidissement de granulés,
- suite au refroidissement, le criblage des granulés de bois finaux dans un dispositif de criblage de granulés,
- suite au criblage, l'échantillonnage automatique des granulés de bois par remplissage automatique d'un cylindre d'échantillonnage avec des granulés de bois, et
- l'analyse de l'échantillon relativement à sa teneur en humidité par utilisation d'un ou de plusieurs capteurs à micro-ondes, relativement à sa température par utilisation d'un capteur de température et relativement à sa densité en vrac par agencement du cylindre d'échantillonnage au niveau d'une cellule de mesure destinée à mesurer le poids volumétrique de l'échantillon, l'échantillonnage, la mesure de contrôle de l'échantillon relativement à des paramètres de contrôle prédéfinis, l'évaluation de résultats de test, l'ajustement éventuel de paramètres de contrôle appropriés en fonction des résultats de test étant réalisés selon une fréquence prédéterminée et entièrement automatisés lors du fonctionnement de l'installation dans le but de contrôler le niveau de qualité des granulés de bois.

2. Procédé selon la revendication 1, **caractérisé en ce qu'**un point de consigne pour chaque paramètre de contrôle est ajustable afin de contrôler la qualité des granulés de bois.

3. Procédé selon la revendication 1 ou 2, **caractérisé en ce que** la fréquence d'échantillonnage est ajustable.

4. Procédé selon l'une quelconque des revendications précédentes, **caractérisé en ce que** la cellule de mesure délivre un signal de sortie qui dépend du poids de l'échantillon.

5. Procédé selon l'une quelconque des revendications précédentes, **caractérisé en ce que** la surveillance, l'échantillonnage, la mesure de contrôle et l'ajustement de paramètres de contrôle s'effectuent au moyen d'un logiciel d'ordinateur.

6. Procédé selon la revendication 5, **caractérisé en ce que** le logiciel d'ordinateur est piloté à distance.

7. Procédé selon l'une quelconque des revendications précédentes, **caractérisé en ce que** la documentation relative à la qualité des granulés s'effectue automatiquement au cours de la mesure de contrôle afin de garantir la conformité des granulés de bois fabriqués à la qualité recherchée.
